# EUROPEAN PATENT APPLICATION

(11) **EP 2 460 532 A1**
(43) Date of publication of application: **06.06.2012**
(21) Application number: 10803747.4
(22) Date of filing: 14.06.2010
(51) Int. Cl.: A61K 38/48, A61P 15/16

(54) **CHEMICAL METHOD FOR SEXUAL STERILISATION AND LIBIDO SUPPRESSION IN MALE MAMMALS**

(30) Priority: 28.07.2009 BR PI0902699
(71) Applicant: Vivacqua, Marcelo, Cep: 29314-100 (BR)
(72) Inventor: Vivacqua, Marcelo, Cep: 29314-100 (BR)
(74) Representative: Clyde-Watson, Zöe
(86) International application number: PCT/BR2010/000207
(87) International publication number: WO 2011/011843

(57) **Abstract**

A chemical method for sexual sterilisation and libido suppression in male mammals is indicated to achieve the biological sterilisation of male mammals such as bovines, sheep, goats, equines, swine, dogs, cats and humans. The invention consists in a sterile aqueous solution containing an active sclerosing principle (lactic acid) and an enzyme (papain). The enzyme causes digestion of testicle tissue, intensifying the acid's necrosing effect and causing it to act faster and more efficiently. The association of these two active principles provokes an acute inflammatory reaction with a quick resolution **characterised by** fibrosing of the organ. This effect leads to the loss of the gametogenic and androgenic functions, and causes sterility and libido suppression, respectively. The biological sterilisation process can be easily carried out and does not require additional treatments, requiring less handling of the animals, with less stress and increased weight gain. The process is not painful either during or after administration, owing to the lesion of the testicle nerve endings at the moment of application, and to the fact that the greatest number of sensitive structures is located in the scrotal sac. The meat of thus treated cattle does not require for consumption a waiting period between application and slaughter, since the biological active principles can be easily metabolised by the animal organism. This method has already been successfully used in more than 3,000 animals, and is the only method available today in which naturally available biological active principles are used.

## Description

The present invention refers to a non-therapeutic biological method of treatment that aims the sexual sterilization and elimination of libido, and is indicated for male mammals, such as cattle, sheep, goats, dogs, domestic cats, horses, pigs and man, which have testicles in scrotum. The method is based on an injection of a sterile aqueous solution containing active ingredients with sclerosing (lactic acid, 20-70%) and enzymatic (papain, 5-20%) functions, administered to the cranial portion of the testicle, resulting in organ fibrosis with loss of gametogenic and androgenic function.

Orchiectomy (removal of the testicles) or castration of male ruminants is necessary for several reasons, mainly decreasing aggressiveness, easing handling and reducing the risk of injury to humans and other animals; more time dedicated to feeding, resulting in greater weight gain; avoiding the risk of unwanted crossbreed by low genetic potential males; and provide better quality carcasses, due to the accumulation of fat and greater rate of prime meat when compared to integer animals. The methods commonly used in the process of castration are based, in most cases, on a surgery, that is, the removal of the testicles by emasculation, using instruments that crush the spermatic cord, resulting in the interruption of testicular blood flow to promote atrophy of the organ.

Another method suitable for carrying out sterilization and elimination of libido is the chemical method. In the 1960s, studies were initiated in order to use sclerosing substances injected directly in the testicles or spermatic cord, with the purpose of promoting the total loss of the function thereof (sperm and androgen hormones production). Chemical sterilization was attempted in monkeys, hamsters, rabbits, rats and dogs by intratesticular administration of some agents such as ferrous chloride (Kar et al. 1965), danazol (Dixit et al., 1975), BCG (Das et al. 1982), tannic zinc, (Fahim et al., 1982), glycerol (Weinbauer et al., 1985, Immegart 2000), glucose, NaCl (Heath et al., 1987, Russell et al 1987.) DBCP (Shemi et al. 1988 ), lactic acid (Fordyce et al. 1989), zinc arginine (Fahim et al. 1993), sodium fluoride (Sprando et al. 1996), formalin (Balar et al. 2002) and calcium chloride (Samanta 1998, Jana et al . 2002), potassium permanganate acetic glacial (Giri et al. 2002). In ruminants males intratesticular injection of lactic acid (Hill et al. 1985), tannic acid, zinc sulfate (Feher et al. 1985) alfahidroxipropionico acid (Cohen et al. 1995), formalin (Ijaz et al. 2000) CastrateQuin 14 (Soerensen et al. 2001) were used. None of the methods proposed for the chemical castration was successful, since none of the products tested was effective to achieve its purposes. The most common problems observed in this process were the return of libido in animals subjected to administration and an uncontrollable process of testicular tissue necrosis, leading to secondary infection and a systemic commitment, weight loss and even death.

Regarding the surgical procedure, it is usually performed by unauthorized people, without the use of anesthetics and without aseptic procedures, often resulting in complications such as hemorrhagic processes, infection, myiasis, tetanus, among others, which usually culminates in the appearance of sequelae that impair the development of animal and in some cases may even lead it to death.

The use of emasculator requires an adequate training of the operator, since, otherwise, complications that may culminate with the need for a surgical intervention and may result in the death of the animal can occur.

In addition to the above economic losses arising from complications, it should also be noted that these lead to medication and labor expenses with nursing procedures. It is also worth noting that none of the methods described above considers animal welfare, or the pain and suffering that these processes can cause to animals. Besides the humanitarian side, which is the main, and the non-observance of the cares which aim to minimize pain and suffering, there is the fact that the stress caused by these situations leads to decreased immunity of the livestock animals, with consequent reduction of its productive rates (weight gain, feed conversion), and make them more susceptible to illness.

The present invention aims to provide a method of castration biologically safe for the animal and operator, which will overcome the problems presented by animals subjected to the prior art processes. The method consists in the intratesticular administration of a sclerosing solution comprising biological active ingredients (papain extracted from papaya and lactic acid extracted from milk), that once done, promote an inflammatory reaction with subsequent fibrosis and elimination of gametogenic and androgenic functions, resulting in sterilization and loss of sexual libido.

The invention is based on the association of 2 to 10% papain enzyme with a lactic acid solution in a sterile aqueous solution at a concentration of 20 to 80%, that once administered to the testicles, triggers a more acute inflammatory reaction with faster resolution, resulting in a greater efficiency in the treatment, whereas the enzyme facilitates the diffusion of acid. The anticipation in the resolution of the inflammatory process involves a shorter time of discomfort due to edema in treated animals. Evaluations conducted in order to assess the physiological and behavioral parameters that characterize pain during and immediately after intratesticular administration of the solution, such as changes in cardiac and respiratory frequencies, vocalization, look or lick the administration site and prolonged decumbency, showed that animals do not experienced this sensation. This is probably because the administered active ingredients cause irreversible lesion in nerve structures of the testicles structures leading to the interruption the transmission of any neurogenic stimulus to the central nervous system. Additionally, it was already demonstrated by several authors that the testicular parenchyma is poorly innervated; these nerve structures are concentrated in the scrotum. The testicular fibrosis, the final stage of the process, leads to gametogenic and androgenic function loss. Parallel to this, the animals which received the recommended dose did not show adverse systemic (fever, decreased appetite and weight loss) neither local (hyperthermia, abscess and necrosis) reactions.

One of the great advantages of the disclosed method lies on the fact that, since it is a "closed" procedure, the chances of environmental contamination are virtually null, which allows it to be carried out independently of the year season (rainy season) or the conditions of pastures (flooded areas or with plants that may cause irritation or bleeding in the surgical wound), unlike the surgical method, that can trigger infections, tetanus and myiasis; which may result in spending on medicines, manpower and weight loss in the affected animals.

The administration of sterilizing biological solution should be performed after prior asepsis of the scrotum, being applied a volume ranging from 0.2 (cats) to 20 ml (cattle and horses) in each testicle, at its cranial portion, not requiring a great skill of the technician who carries out the procedure, requiring only a short demonstrative training to the operator. There is no fixed correlation between the species, body weight, testicular diameter and applied dose, being the same defined by the organ's reaction to it, characterized by contraction of the whole organ during the administration, reaction which indicates that solution reached all body and, therefore, the administration should be discontinued. One caution that should be taken is to prevent the occurrence of solution reflux into the testicular capsule and subcutaneous space, because, if this happens, there will occur pain due to the sterilizing solution contact with nerve structures present in this region, as well as the possibility of a skin ulceration. It should be noted that, if this happens, the goal of the procedure which is to promote the sterilization will not be compromised. The inconvenience caused by technical error, despite does not compromise the main result, should be avoided, because if it occurs, two of the main advantages of the method will eliminated, namely the absence of pain and the need for additional care after the procedure.

The product proved to be innocuous to humans, not having caused irritations, blemishes, pruritus or other undesirable reaction when in contact with the skin of the operator. Due to this characteristic, it is not necessary, therefore, to use of gloves or mask, since the product does not have strong odor that can cause any respiratory discomfort. It is recommended washing hands after administration, the aqueous vehicle leads to low absorption degree and, if this happen, both active ingredients have not been reported to cause intoxications in humans. Lactic acid is a physiological product present in the body of mammals, being a metabolite of glycogenolysis, papain only become harmful if it was inhaled as a powder in large quantities, resulting in pulmonary diseases. Also in relation to the latter, it is used in edible products, such as meat softeners, and is therefore well tolerated by human body when ingested in small quantities.

The stability studies showed that when the solution is maintained in amber bottles and kept at room temperature, retains its biological action by more than 30 months, the utmost period which the solution was tested.

Experiments were performed with 80 animals, in which one half was treated with the chemical solution and the other subjected to the surgical castration process. It was observed that 20% of the animals showed discomfort signals, with movements and prolonged decumbency after the administration. These signals disappeared in 8 hours. At no time during the first 24 hours, the animals showed signals that represented pain as vocalization, licking the administration site or systemic signals of hyperthermia or decreased appetite.

The testicular swelling was height within 48 hours from administration, receding in 60 days. In the first 24 hours after administration, the animals no longer showed signals of libido or aggression. On the other hand, the animals which underwent surgery had a longer period of suffering until the wound healing, about 15 to 20 days, whereas some were affected by myiasis and infection, requiring returning to the handling centers to be submitted to nursing procedures, such as administration of local and systemic antibiotics and repellents, these procedures result in more stressful situations. None of the treated animals showed returning of libido, so there was no need for a second administration of the drug.

After six months of the experiment, the biologically treated animals showed greater weight gain than the animals which underwent surgery, and it was not observed statistically significant differences after slaughtering in carcass yield, fat cover and front-rear relationship.

Tests were also conducted by sampling for determination of testosterone and semen collection. In all the tests we observed a sharp drop in testosterone levels and azoospermy (no sperm in ejaculate) which indicated 100% efficiency of the drug.

It was made a collection of random fragments of organs, and after histopathological examination it was found no damage in heart, lung, liver and kidney tissues, which proves the safety of the product to animals at the recommended dose. In contrast, the testis samples analyzed showed a process of focal necrosis with total loss of sperm and testosterone production function, due to the destruction of the production sites seminiferous tubules and Sertoli cells, respectively. It is important to point out that this loss of function was not restricted to areas where focal necrosis took place, but throughout the testicular parenchyma, including apparently normal areas at macroscopic examination.

These results were expected, since the mammalian organism has the ability to metabolize the biological active ingredients without undergoing any toxic reaction.

The recommended period for proceeding with the slaughter of treated animals is at least 12 months after administration. In the above-reported experiment, the slaughtering was earlier due to operational purposes of the enterprise where it was carried out. It is estimated that by extending such period, the slaughterhouse rate gains tend to be higher than those observed in animals treated with the surgical procedure. It must be highlighted that, while the animals which underwent surgical procedures were more susceptible to complications, as already mentioned, requiring a return to handling centers to receive nursing care, those which underwent the biological process of the present invention returned to the handling center only for weighing and assessing their testicles and general conditions. Under normal conditions, the biologically treated animals do not need to return to the handling centers, resulting in less stress, consequently increasing the feeding rate and weight gain.

In addition to the animals tested in this experiment, the biological sterilizing solution was tested in approximately 2000 cattle, 80 sheep, 70 goats, 10 pigs, 15 horses, 16 dogs and 8 cats. It was observed the returning of libido in about 5% of treated animals. In these animals, a second administration was made to achieve the desired effect. This procedure is recommended to animals showing signals of libido after 15 days from administration. With regard to equines, literature reports the mating, observed in approximately 30% of the surgically castrated animals as part of a conditioned behavior, having no relation to the testosterone levels. For other species, probably there may have been errors in administering the solution, especially dose or site of administration error in animals which presented returning of libido, given the low incidence.

These findings led to conclude that this invention reached the proposed objective, successfully overcoming the obstacles faced by other formulations suitable for the same use, which failed due to insufficiency, leading to the return of reproductive functions of the treated animals, or otherwise, causing uncontrollable necrotic processes with abscess formation, requiring surgical intervention, drug and labor spending, and affecting the animals health and welfare.

It is important to point out that the only commercially available products in the world suitable for chemical sterilization by intratesticular injection are indicated for use in dogs, which, however, shows no concern with minimizing the suffering caused by the administration and also contains chemical active ingredients, for which the medium and long term effects are not yet known.

The described solution has the advantage of not triggering pain when properly applied, thereby helping to meet international standards designed to protect the animals against procedures that cause pain and, when applied in livestock animals, dispenses the grace period between the administration and consumption of meat from these animals, assuring the safe consumption by humans. This new method discourages farmers to use hormones as an alternative to the risks of the surgical process, providing a product originated from livestock animals that were not subjected to cruel practices of handling, highly valorized in developed and Muslim religion countries. Because the solution comprises a biological active ingredient, biosafety is preserved, since meat contamination does not occur there is no risk environment contamination with any residues present in bodily secretions of treated animals.

## Claims

1. CHEMICAL METHOD FOR SEXUAL STERILIZATION AND LIBIDO ELIMINATION IN MALE MAMMALS, being a process designed to carry out sterilization and elimination of sexual libido in male mammals with external testicles by intratesticular injection of a sterile aqueous solution of a sclerosing lactic acid concentration from 20 to 70%, for the purpose of triggering an acute inflammatory process, followed by fibrosis of the testicular parenchyma, resulting in disruption of spermatogenesis (sperm production), leading to loss of fertility, and androgen (synthesis of male hormones), which causes loss of libido **characterized by** the addition of 2-10% papain enzyme to the lactic acid solution, which promotes an acceleration of the inflammatory process caused by tissue digestion, anticipating the resolution thereof and consequently of the testicular fibrosis reaction, minimizing the discomfort time of the treated animal.

2. CHEMICAL METHOD FOR SEXUAL STERILIZATION AND LIBIDO ELIMINATION IN MALE MAMMALS, according to claim 1, **characterized by** the injection of a sclerosing solution in the cranial portion of the testicle near the head of the epididymis, favoring the spread of solution in the testicular parenchyma, preventing the return of libido due to incomplete action of the product or an uncontrolled necrosis with abscess formation process.

3. CHEMICAL METHOD FOR STERILIZATION AND DISPOSAL OF SEXUAL LIBIDO IN MALE MAMMALS, according to claim 1, **characterized by** the use of sclerosing solution to promote sterilization and elimination of sexual libido in male mammals of bovine, ovine, caprine, equine, porcine, canine, feline and human species.
